# EUROPÄISCHE PATENTSCHRIFT

(11) **EP 1 805 278 B1**
(45) Veröffentlichungstag und Bekanntmachung des Hinweises auf die Patenterteilung: **06.01.2010**
(21) Anmeldenummer: 05800484.7
(22) Anmeldetag: 18.10.2005
(51) Int. Cl.: C09K 9/02, C07D 498/04, C07D 493/04, C07D 311/92, C07D 311/94, G02B 5/23, B29D 11/00

(54) **PHOTOCHROME H-ANNELLIERTE BENZO( F)CHROMEN-DERIVATE**
PHOTOCHROMIC H-ANNELLATED BENZO(F)CHROME-DERIVATIVES
DERIVES PHOTOCHROMES DE BENZO(F)CHROMENES H-ANNELES

(30) Priorität: 21.10.2004 DE 102004051509
(43) Veröffentlichungstag der Anmeldung: 11.07.2007
(73) Patentinhaber: Rodenstock GmbH, 80469 München (DE)
(72) Erfinder: MELZIG, Manfred, 82234 Wessling (DE); ROHLFING, Yven, 81547 München (DE); WEIGAND, Udo, 80638 München (DE)
(74) Vertreter: Müller-Boré & Partner Patentanwälte
(86) Internationale Anmeldenummer: PCT/EP2005/011202
(87) Internationale Veröffentlichungsnummer: WO 2006/045495

(56) Entgegenhaltungen:
- WO-A-01/36406
- WO-A-02/22594
- WO-A-99/15518
- US-A- 5 869 658

## Beschreibung

Die vorliegende Erfindung betrifft spezifische photochrome h-annellierte Benzo[f]chromen-Derivate sowie deren Verwendung in Kunststoffen aller Art, insbesondere für ophthalmische Zwecke. Insbesondere betrifft die vorliegende Erfindung von Benzo[f]chromenen abgeleitete photochrome Verbindungen, die in der geschlossenen Form besonders langwellige Absorptionsmaxima bei gleichzeitig guter Leistungsfähigkeit in der offenen, farbigen Form aufweisen, wodurch sie bei Anwendung in phototropen Gläsern mit den darin verbreitet eingesetzten Indenonaphthopyranen gut harmonieren.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, dass diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Der Weltmarkt für photochrome Brillengläsern, sowohl aus Silikat wie aus Kunststoff, wird von den Farben grau und braun beherrscht. Färbungen wie grün, blau, magenta, orange oder gelb spielen eine völlig untergeordnete Rolle.

Bei allen derzeit auf dem Markt befindlichen photochromen Kunststoffgläsern werden diese beiden Farben durch Mischungen von mindestens zwei photochromen Farbstoffen erzielt. Diese können, wie in US 6,306,3126 dargelegt, in zwei Gruppen eingeteilt werden, nämlich in solche, deren längstwelliges Absorptionsmaximum oberhalb von 550 nm liegt, die also im angeregten Zustand eine violette, blaue bis grüne Transmissionsfarbe ergeben, und in solche, deren längstwelliges Absorptionsmaximum unterhalb 550 nm liegt. Deren Transmissionsfarbe reicht von gelb über orange bis rot.

Zur ersten Gruppe zählen von 1-Naphtholen abgeleitete 2H-Naphthopyrane und ihre davon durch Annellierung abgeleiteten höheren analogen Derivate. Diese sind beispielsweise in US 5,698,141, US 5,723,072, US 6,146,554, US 6,225,466, US 6,331,625 bzw. US 6,340,765 beschrieben. Zu anderen Klassen gehörende photochrome Verbindungen, wie in US 4,931,220 oder EP 0 600 688 beschrieben, weisen zwar Absorptionsmaxima oberhalb von 550 nm auf, sind aber wegen ihrer geringen Lebensdauer bzw. der geringen Bandbreite der langwelligen Absorption nicht mehr im kommerziellen Einsatz. Die langwellig absorbierenden photochromen Farbstoffe aller derzeit auf dem Markt erhältlichen grauen oder braunen photochromen Kunststoffgläser (z.B. Rodenstock Perfalit ColorMatic Extra^{®} - seit 1999 -, Transitions Next Generation^{®} - seit 2002 -, Hoya Solio^{®} 1,55 - seit 2004) gehören zu den vorstehend von 1-Naphtholen abgeleiteten Verbindungen.

Zur zweiten Gruppe gehörende Farbstoffe sind meist in 2-Stellung Aryl- oder Heteroaryl substituierte 3H-Benzo- bzw. 3H-Napthopyrane, welche von 2-Naphtholen abgeleitet sind, wie in US 5,244,602, US 5,427,774, US 5,552,090, US 5,552,091, US 5,585,042 und WO 97/20239 beschrieben. Auch die in US 4,826,977 beschriebenen Spiroadamantan-substituierten Verbindungen gehören in diese Gruppe. Von 1-Naphtholen abgeleitete 2H-Naphtho[1,2-b]pyrane sind nur verwendbar, wenn die offene Form durch Substitution in 5-Stellung des Systems sterisch gehindert ist, wie in EP 1 248 778 beschrieben. Ohne diese Hinderung ist die Aufhellung für die Verwendung in Brillengläsern zu langsam.

Im Handel erhältliche photochrome Verbindungen, wie Reversacol Sunflower, Corn Yellow, Flame und Ruby (James Robinson) oder CNN-4 und CNN-8 (Tokuyama Soda) sind in EP 0 691 965 und US 6,719,926 beschrieben. Diese Verbindungen besitzen alle in 6-Stellung des Naphthopyransystems eine Aminogruppe, zumeist Piperidin oder Morpholin, die eine sehr hohe molare Extinktion (IOD > 1,5) im Absorptionsmaximum ergibt. Ohne diese funktionale Gruppe liegt der Wert um etwa 1,5 niedriger. Leider hat dieses stark polare Substitutionsmuster eine ausgeprägte Solvatochromie zur Folge, so dass ein Teil des eingesetzten Farbstoffs in der festen Lösung (Kunststoffmatrix) in der offenen Form vorliegt. Dies zeigt sich in leicht farbigen Gläsern auch unter völligem Ausschluss von Anregungslicht. Zudem ist auch die Transmission nach V_{λ} um 4-10% herabgesetzt. Beispielhaft seien hier vor allem die braunen Varianten von Rodenstock ColorMatic Extra^{®} und Hoya Solio^{®} 1,55 genannt.

Ein weiterer Nachteil ist die im Vergleich zu den Farbstoffen der ersten Gruppe um 20-25 nm hypsochrom verschobene Absorption der geschlossenen Form. Die Abstimmung der Komposition, d.h. die jeweiligen Konzentrationen der eingesetzten photochromen Farbstoffe, zur Erzielung einer grauen oder braunen Farbe erfolgt derart, dass die Wunschfarbe im normalen direkten oder indirekten Sonnenlicht erreicht wird. Wird der ganz kurzwellige Anteil des sichtbaren Sonnenlichts (380-400 nm) selektiv gefiltert oder geblockt, z.B. durch wärmedämmend beschichtetes Fensterglas oder Verbundglas in Kraftfahrzeugen, so nehmen die Gläser im Falle eines grauen Glases eine blaue bzw. im Falle eines braunen Glases eine graue Farbe an. Dies kann in taghellen Räumen bei den heute am Markt erhältlichen photochromen Kunststoffgläsern gut beobachtet werden und ist ein kosmetischer Nachteil.

In WO 02/22594 sind Verbindungen beschrieben, die aufgrund ihrer Struktur zwar eine längerwellige Absorption der geschlossenen Form aufweisen. Der wesentliche Aspekt liegt hier jedoch in der Bereitstellung leistungsstarker langwellig absorbierender, also violetter bis blauer Farbstoffe, d.h. die eine längerwellige Absorption der offenen Form aufweisen. Dies wurde durch die Einführung von Aminosubstituenten in das Naphthopyransystem erreicht. Diese Verbindungen weisen längstwellige Absorptionsmaxima der offenen Form auf, die nicht unterhalb 540 nm liegen. Ein Nachteil ist jedoch auch hier wieder die Vorfärbung bei der Verwendung in Brillenglas-Kunststoffmaterialien.

US 5,869,658 beschreibt Verbindungen ähnlicher Grundstruktur, deren offene Form im gewünschten Spektralbereich absorbiert. In dieser Schrift werden jedoch nur Indeno-annellierte Naphtho[2,1-b]pyrane behandelt, die zudem in 6-Stellung durch eine Alkoxygruppe substituiert sind. Dies ist durch die Synthese zwingend notwendig, da der Ringschluss ohne einen aktivierenden Substituenten, der die Verknüpfungsstelle in p-Position nucleophil macht, nicht stattfindet. Größere annellierte Ringsysteme als der 5-Ring sind nicht beschrieben und auf diesem Weg nicht möglich. Zudem sind die Aufhellgeschwindigkeiten sehr hoch. Dies führt bei Belichtung im Gleichgewicht nur zu einem geringen Anteil offener, d.h. farbiger Moleküle. Die Eindunkelungsleistung ist gering (ΔOD < 0,5). Ebenso ist die Absorption der geschlossenen Form mit Maxima zumeist unter 370 nm deutlich kürzerwellig als die der Verbindungen der ersten Gruppe. Dies ist jedoch nicht ausreichend, den langwelligen UV-Anteil des Sonnenlichts auszunutzen. Die in US 5,869,658 offenbarten Farbstoffmoleküle sind relativ planar, da die Abstoßung der H-Atome (in 8- und 9-Stellung des Formelbilds in Spalte 21) nicht sehr stark ist. Das Beispiel 3 fällt aus dem Rahmen, da hier die langsame Aufhellung und damit stärkere Eindunkelung ΔOD durch einen Fluor-Substituenten in 2-Stellung an den Phenylringen B bzw. B' erzwungen wird. Diese Wirkung, etwa eine Vervierfachung des ΔOD Werts, ist bereits in US 5,066,818 beschrieben. Diese die freie Rotation behindernde Substitution führt jedoch zu einer unerwünscht starken Abhängigkeit der Aufhellung von der das Molekül umgebenden Matrix, d.h. einer sehr breiten Verteilung der Aufhellgeschwindigkeit, wenn das Molekülensemble in einer Matrix betrachtet wird, bzw. einer stark unterschiedlichen Aufhellgeschwindigkeit in verschiedenen Kunststoffmaterialien.

EP 1 230 234 beschreibt ebenfalls 2H-Diarylnaphthopyrane, die mit ankondensierten Ringen substituiert sind. Die Kondensation mit einem Indenring in der gezeigten Struktur führt zu Verbindungen, die aufgrund der nicht vorhandenen sterischen Hinderung zwischen der CH₂-Gruppe des 5-Rings und dem H-Atom in 8-Stellung meist so schnell wie die Vergleichsverbindung C5 aufhellen. Verbindungen dieser Struktur sind, wie in US 3,567,605 beschrieben, nur bei sehr tiefen Temperaturen ausreichend photochrom. Auch beim 6-Ring ist die Hinderung noch sehr gering, die Aufhellung schnell und damit die beobachtete Einfärbung nur gering. Zudem ist die Auswahl möglicher Verbindungen sehr gering, die beschriebene Synthese lässt nur die Herstellung in 6- und 7-Stellung mit aktivierenden Gruppen substituierten Verbindungen zu. Bei Verwendung nur einer Methoxygruppe oder weniger aktivierenden höheren Alkoxygruppen findet keine Ringschlussreaktion statt. Größere Alkanringe sind synthetisch möglich, die sterische Hinderung ist jedoch im Vergleich zu den Verbindungen der Struktur gemäß US 5,869,658 oder WO 02/22594 immer gering, ebenso die Eindunkelungsleistung. Der wesentliche Aspekt dieser Verbindungen gemäß EP 1 230 234 war jedoch in erster Linie die Bereitstellung von intrinsisch grauen oder braunen Verbindungen. In der offenen Form steht die (substituierte) Phenylgruppe meta statt para zur Ethylenbrücke, dies führt zu völlig anderem, teilweise sogar kontroversem Verhalten.

Somit liegt der vorliegenden Erfindung die Aufgabe zugrunde, neue photochrome Farbstoffe bereitzustellen, die verbesserte Eigenschaften gegenüber den im Stand der Technik verfügbaren Verbindungen besitzen sollen. Die photochromen Verbindungen sollen sich gegenüber vergleichbaren Verbindungen aus dem Stand der Technik insbesondere durch eine im nicht angeregten Zustand längerwellige Absorption, d.h. im Bereich von zwischen etwa 380 bis 400 nm, bei gleichzeitig guter Leistungsfähigkeit in der offenen Form, d.h. bei Belichtung durch eine höhere molare Extinktion der angeregten Form, sowie durch gute Kinetik- und Lebensdauereigenschaften auszeichnen, d.h. mit schneller Aufhellungsgeschwindigkeit, die den üblicherweise in phototropen Gläsern gleichzeitig eingesetzten langweilig absorbierenden Verbindungen angepasst ist, sowie gutem Verhalten im Lebensdauertest.

Diese Aufgabe wird durch die in den Ansprüchen gekennzeichneten Gegenstände gelöst.

Insbesondere werden photochrome h-annellierte Benzo[f]chromene mit der allgemeinen Formel (I) bereitgestellt: 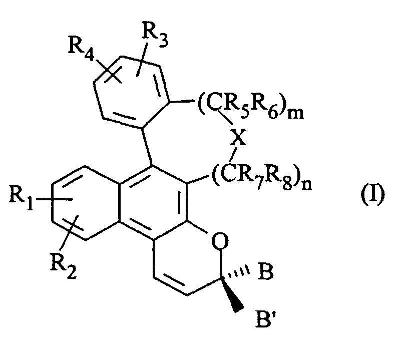 worin
n und m unabhängig voneinander 0, 1 oder 2 bedeuten,
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S aufweisen kann, einem (C₁-C₆)-Alkoxyrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können;
der Gruppe γ, worin die Reste R₁ und R₂ bzw. R₃ und R₄ jeweils eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann;
die Reste R₅, R₆, R₇ und R₈ jeweils unabhängig voneinander aus der Gruppe α und Phenyl ausgewählt sind oder die Reste R₅ und R₆ zusammen mit dem Rest R₃ des direkt benachbarten Benzoringes einen daran annellierten, un-, mono- oder disubstituierten Benzo- oder Pyridoring bilden, dessen Substituenten aus der Gruppe α und Phenyl ausgewählt sein können, oder, wenn m bzw. n 2 bedeuten, die direkt benachbarten Reste R₅ und R₆ zweier benachbarter CR₅R₆-Einheiten bzw. die direkt benachbarten Reste R₇ und R₈ zweier benachbarter CR₇A₈-Einheiten miteinander einen annellierten, un-, mono- oder disubstituierten Benzo- oder Pyridoring bilden, dessen Substituenten aus der Gruppe α und Phenyl ausgewählt sein können, oder die Reste R₅ und R₆ und/oder die Reste R₇ und R₈ zusammen einen (C₃-C₇)-Cycloalkylrest darstellen, der ein oder mehrere Heteroatome, wie beispielsweise Sauerstoff oder Schwefel, aufweisen kann, wobei an diesen Cycloalkylrest ein Benzoring annelliert sein kann;

X aus O, S oder CR₉R₁₀ ausgewählt ist, wobei die Reste R₉ und R₁₀ unabhängig voneinander aus der Gruppe α und Phenyl ausgewählt sind oder die Reste R₉ und R₁₀ zusammen einen (C₃-C₇)-Cycloalkylrest darstellen, der ein oder mehrere Heteroatome, wie beispielsweise Sauerstoff oder Schwefel, aufweisen kann, oder die Reste R₉ und R₁₀ zusammen mit den Resten R₅ und R₆ bzw. R₇ und R₈ einer direkt benachbarten CR₅R₆-Einheit bzw. CR₇R₈-Einheit für einen an die X-C(R₅R₆) bzw. X-C(R₇R₈)-Bindung annellierten, un-, mono- oder disubstituierten Benzo- oder Pyridoring stehen können, dessen Substituenten aus der Gruppe α und Phenyl ausgewählt sein können; mit der Maßgabe, dass X nicht CR₉R₁₀ sein kann, wenn m und n beide 0 sind;

B und B' unabhängig voneinander aus einer der folgenden Gruppen a), b), c) oder d) ausgewählt sind, wobei
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Dibenzofuranyl, Thienyl, Benzothien-2-yl, Benzothien-3-yl, oder Dibenzothienyl ist;
   wobei die Substituenten der Aryl- oder Heteroarylreste in a) und b) solche sind, ausgewählt aus den Gruppen α, β oder γ oder einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, einer N-Morpholingruppe, einer N-Thiomorpholingruppe, einer N-Piperidingruppe, einer N-Azacycloheptangruppe, einer N-Piperazingruppe, einer N-(N'-(C₁-C₆-Alkyl)piperazingruppe, einer N-Pyrrolidingruppe, einer N-Imidazolidingruppe, einer N-Pyrazolidingruppe, einer N-Aziridingruppe, einer N-Azetidingruppe, einer N-Indolingruppe, einer N-Carbazolgruppe, einer N-Phenothiazingruppe, einer N-Phenazingruppe, einer N-Phenoxazingruppe, einer N-Tetrahydrochinolingruppe oder einer N-Tetrahydroisochinolingruppe, wobei die Substituenten vorzugsweise solche aus den Gruppen α und β sind;
c) Struktureinheiten mit den folgenden Formeln (V) und (W) sind: 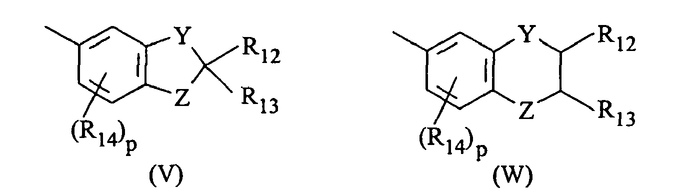 worin
   Y und Z unabhängig voneinander für O, S, CH₂, CMe₂, NH, NPh oder N(C₁-C₆)-Alkyl stehen, die Reste R₁₂ und R₁₃ unabhängig voneinander Wasserstoff oder einen (C₁-C₆)-Alkylrest darstellen und der Rest R₁₄ ein Substituent aus der Gruppe α ist, wobei p 1, 2, oder 3 ist,
   oder
d) B und B' miteinander einen un-, mono- oder disubstituierten 9,10-Dihydroanthracen-, Fluoren-, Thioxanthen- oder Xanthen-9-ylidenrest, Benzo[b]flouren-11-ylidenrest, 5H-Dibenzo[a,c]cyclohepten-, Dibenzosuberon- oder 5H-Dibenzo[a,c]cyclooctan-5-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Substituenten der ungesättigten Cyclen aus der Gruppe α ausgewählt sind.

Gemäß der vorliegenden Erfindung werden durch h-Annellierung von Benzo[f]chromen-Systemen Verbindungen bereitgestellt, deren photochrome Eigenschaften gegenüber den im Stand der Technik bekannten Verbindungen wichtige Vorzüge aufweisen. Insbesondere weisen die erfindungsgemäßen Verbindungen langwellige Absorptionsmaxima in der geschlossenen (farblosen) Form bei gleichzeitig guter Leistungsfähigkeit in der offenen Form, d.h. bei Belichtung eine höhere molare Extinktion der angeregten Form, sowie gute Kinetik- und Lebensdauereigenschaften auf. Zudem zeigen die erfindungsgemäßen photochromen h-annellierten Benzo[f]chromen-Derivate gegenüber entsprechenden Verbindungen aus dem Stand der Technik vergleichbar gute Lebensdauer- bzw. bessere Kinetikeigenschaften, d.h. eine schnelle, den heute üblicherweise in phototropen Gläsern gleichzeitig verwendeten langwellig absorbierenden photochromen Farbstoffen angepasste Aufhellungsgeschwindigkeit sowie gutes Verhalten im Lebensdauertest.

Der in h-Position des Benzo[f]chromen-Systems annellierte Cyclus bzw. Heterocyclus ist vorzugsweise ein Fünfring (n = m = 0; vgl. nachstehende Formel (II)), Sechsring (n = 1, m = 0 bzw. m = 1, n = 0; vgl. nachstehende Formel (III)) oder Siebenring (vorzugsweise mit n = m = 1; vgl. nachstehende Formel (IV)).

Das Fünfringsystem mit einer CH₂-Brücke ist nahezu planar, aber stark verspannt. Mit dem Standard-Molekülgeometrie-Programm Hyperchem 7 (Monte Carlo, Mm+, 100 Zyklen) ergibt sich für den Winkel ε (Verdrehung des Phenylrings gegen die Naphthalinringebene) der Wert -0,27°. Die Einführung eines Sauerstoffatoms entspannt das Molekül durch Verdrehung des Winkels ε auf -27,25°. In gleicher Weise wirkt der Ersatz der CH₂-Brücke durch eine CH₂-CH₂-Brücke zum Sechsring (ε = -25,39°). Der Siebenring mit einer CH₂-CH₂-CH₂-Brücke führt bereits zu einer helicen-ähnlichen Struktur (ε = -49,25°). Der 5-Ring mit O oder S hat deutlich stärkeren aromatischen Charakter als mit einer CH₂- oder CR₂-Gruppe. Dies führt zu einer bathochromen Verschiebung der Absorption.

Bevorzugte photochrome h-annellierte Benzo[f]chromen-Derivate gemäß der vorliegenden Erfindung weisen die nachfolgenden allgemeinen Formeln (II), (III) bzw. (IV) auf: 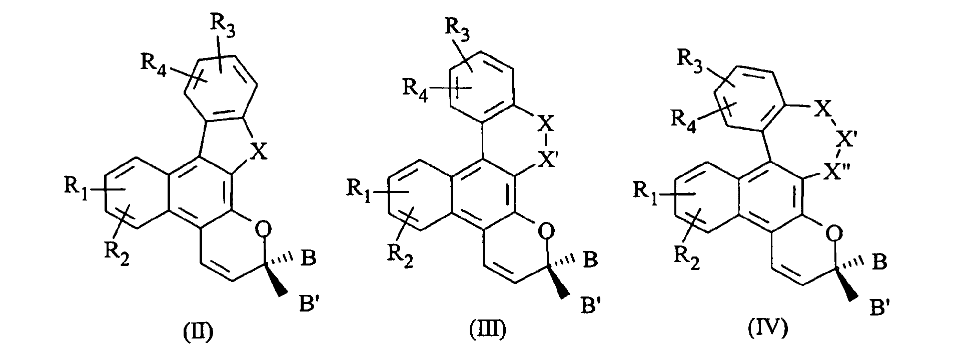 worin B, B', R₁, R₂, R₃ und R₄ wie vorgenannt definiert sind und
in Formel (II) X = O oder S ist;
in Formel (III) X und X' unabhängig voneinander aus O, S oder CR₉R₁₀ ausgewählt sind, mit der Maßgabe, dass mindestens eines von beiden CR₉R₁₀ ist; bzw. in Formel (IV) X, X' und X" unabhängig voneinander aus O, S oder CR₉R₁₀ ausgewählt sind, mit der Maßgabe, dass, wenn X' = O oder S ist, X und X" = CR₉R₁₀ sind.

Die Reste R₅, R₆, R₇ und R₈ werden jeweils unabhängig voneinander vorzugsweise aus der Gruppe α ausgewählt. Wenn in den Formeln (III) bzw. (IV) X, X' bzw. X" für CR₉R₁₀ steht, können die Reste R₉ und R₁₀ miteinander insbesondere für einen (C₁-C₆)-Alkylrest oder (C₃-C₇)-Cycloalkylrest stehen, der ein oder mehrere Heteroatome aufweisen kann.

Die Reste R₁ und R₂ bzw. R₃ und R₄ können jeweils eine an den aromatischen Ring gebundene -A-(CH₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; als -A-(CH₂)ₖ-D- Einheit kann insbesondere -O-(CH₂)₂-O-angeführt werden, wobei gegebenfalls an die Ethylengruppe davon ein Benzocyclus annelliert ist. Die -A-(CH₂)ₖ-D- Einheit ist dabei über A und D in ortho-Stellung zueinander an den jeweiligen Benzoring gebunden.

In einer besonders bevorzugten Ausführungsform sind in den vorstehend aufgeführten Formeln (I), (II), (III) bzw. (IV) B und B' unabhängig voneinander mono-, di- oder trisubstituierte Arylreste, wobei der Arylrest jeweils ein Phenylrest oder ein Naphthylrest ist.

Besonders bevorzugte photochrome h-annellierte Benzo[f]chromen-Derivate gemäß der vorliegenden Erfindung sind:
(1) 2-(4-Methoxyphenyl)-2-phenyl-2H-benzofurano[1,2-h]benzo[f]chromen,
(2) 2-(4-Methoxyphenyl)-2-phenyl-2H-benzothiopheno[1,2-h]benzo[f]chromen,
(3) 2-(4-Methoxyphenyl)-2-phenyl-2H-13,14-dihydro-naphtho[1,2h]-benzo[f]chromen,
(4) 7-Methoxy-2-(4-methoxyphenyl)-2-phenyl-2H-13,14-dihydro-naphtho[1,2-h]-benzo[f]chromen,
(5) 11-Methoxy-2-(4-methoxyphenyl)-2-phenyl-2H-13,14-dihydro-naphtho[1,2-h]-benzo[f]chromen,
(6) 2-(4-Methoxyphenyl)-2-phenyl-2H,13H-chromeno[1,2-h]benzo[f]chromen,
(7) 2-(4-Methoxyphenyl)-2-phenyl-2H,14H-chromeno[1,2-h]benzo[f]chromen,
(8) 7-Methoxy-2-(4-methoxyphenyl)-2-phenyl-2H-benzocycloheptano-[1,2-h]-benzo[f]chromen

Die längstwelligen Absorptionsmaxima λₘₐₓ der geschlossenen (farblosen) sowie der offenen (farbigen) Form verschiedener Verbindungen sind folgender Tabelle zu entnehmen (die Zahlen beziehen sich auf die Auflistung der besonders bevorzugten Verbindungen). Außerdem ist das Leistungsvermögen der farbigen Form der erfindungsgemäßen Verbindungen angegeben. Dazu wurden jeweils 500 ppm photochromer Farbstoff in die phototrope Matrix von ColorMatic Extra^{®} eingebracht und nach Polymerisation die Transmission definiert an einer Kinetikbank bei 23 °C (15 min belichtet bei 50 klux) gemessen. Je geringer die Transmission, desto intensiver ist die Leistung des photochromen Farbstoffes bei Belichtung. Es muß allerdings noch beachtet werden, daß die Transmission gewichtet zum Augenempfindlichkeitsmaximum V_{λ} gemessen wurde, so daß gelborange Farbstoffe, deren Absorptionsmaximum weiter vom menschlichen Empfindlichkeitsmaximum entfernt ist, eine höhere Transmission aufweisen als orangerote Farbstoffe, deren Absorptionsmaximum näher liegt. Die Intensität zweier photochromer Farbstoffe läßt sich also nur dann gut über den Transmissionsgrad bei Belichtung vergleichen, wenn deren längstwelliges Absorptionsmaximum (offene Form) nicht zu weit voneinander abweichen (ansonsten würden gelbere Farbstoffe scheinbar schwächer eingestuft als rötere). In der letzten Zeile der nachfolgenden Tabelle ist eine Verbindung aus dem Stand der Technik gemäß US 5,869,658 zum Vergleich aufgeführt.

Die Angaben in der nachstehenden Tabelle zu m, n, X, R₁ sowie R₃ beziehen sich auf die Struktur (I):
- B ist jeweils 4-Methoxyphenyl
- B' ist jeweils Phenyl
- R₂ = R₄ ist jeweils H

| Nr. | m R_{5/6} | n R_{7/8} | X | R₁ | R₃ | λₘₐₓ (geschlossen) | λₘₐₓ (offen) | Transmission eingedunkelt |
|---|---|---|---|---|---|---|---|---|
| (1) | 0 | 0 | 0 | H | H | 380 nm | 470 nm | 53% |
| (2) | 0 | 0 | S | H | H | 385 nm | 475 nm | 49% |
| (3) | 1 H/H | 0 - | CH₂ | H | H | 385 nm | 450 nm | 58% |
| (4) | 1 H/H | 0 - | CH₂ | 7-OMe | H | 400 nm | 470 nm | 40% |
| (5) | 1 H/H | 0 - | CH₂ | H | 11-OMe | 390 nm | 455 nm | 55% |
| (6) | 1 H/H | 0 - | O | H | H | 380 nm | 455 nm | 58% |
| (7) | 0 - | 1 H/H | O | H | H | 395 nm | 460 nm | 57% |
| (8) | 1 H/H | 1 H/H | CH₂ | 7-OMe | H | 380 nm | 485 nm | 40% |
| Stand der Technik | 0 | 0 | CH₂ | 6-OMe | H | 380 nm | 465 nm | 65% |

Aus der Tabelle ist ersichtlich, daß die erfindungsgemäßen Verbindungen eine höhere Leistung im eingedunkelten Zustand im Vergleich zu der beispielhaften Verbindung aus dem Stand der Technik aufweisen. Außerdem ist das Absorptionsmaximum der geschlossenen Form in den allermeisten Fällen bathochrom verschoben. Diese Eigenschaft ist besonders wichtig, wenn aufgrund von Streueffekten in der Atmosphäre nur wenig UV-Strahlung zur Anregung zur Verfügung steht. Da längerwellige UV-Strahlung geringer als kürzerwellige gestreut wird, dunkeln diese Farbstoffe im Gegensatz zu kürzerwellig absorbierenden photochromen Farbstoffen auch bei ungünstigeren Verhältnissen noch gut ein.

Die erfindungsgemäßen Verbindungen können in Kunststoffmaterialien bzw. Kunststoffgegenständen jeglicher Art und Form für eine Vielzahl von Einsatzzwekken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Benzo[f]chromen-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzo[f]chromen-Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Benzo[f]chromen-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen h-annellierten Benzo[f]chromen-Derivate beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

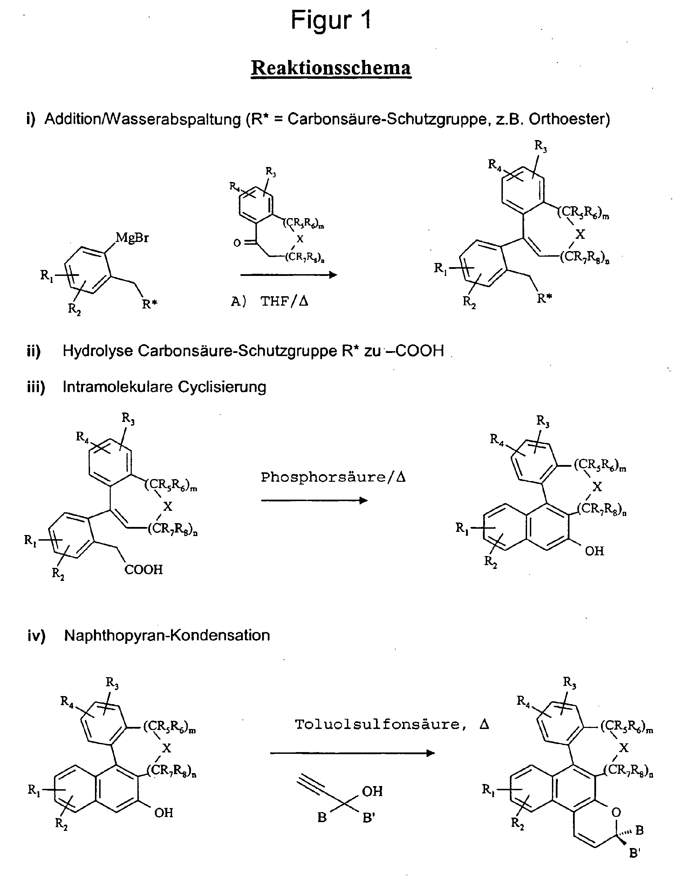

Die erfindungsgemäßen photochromen h-annellierten Benzo[f]chromen-Derivate mit der allgemeinen Formel (I) bzw. (II), (III) und (IV) lassen sich durch Umsetzung von geeignet substituierten annellierten 2-Naphthol-Derivate mit geeignet substituierten 2-Propin-1-ol-Derivaten in bekannter Weise (vgl. WO 02/22594) synthetisieren. Nachfolgend wird die Herstellung der erfindungsgemäßen Verbindungen anhand eines allgemeinen Reaktionsschemas erläutert (siehe Figur 1).

Geeignet substituierte aromatische Grignardverbindungen, die in ortho-Stellung eine geschützte Essigsäure-Funktion aufweisen, werden an cyclische aromatischaliphatische Ketone (Schritt i) addiert. Nach Wasserabspaltung und Entfernung der Carbonsäure-Schutzgruppe entstehen via intramolekularer Cyclisierung substituierte annellierte 2-Naphthol-Derivate (Schritte ii und iii). Anschließend werden diese 2-Naphthol-Derivate mit geeignet substituierten 2-Propin-1-ol-Derivaten gemäß Schritt iv) zu den erfindungsgemäßen Verbindungen umgesetzt.

## Patentansprüche

1. Photochrome h-annellierte Benzo[f]chromene mit der allgemeinen Formel 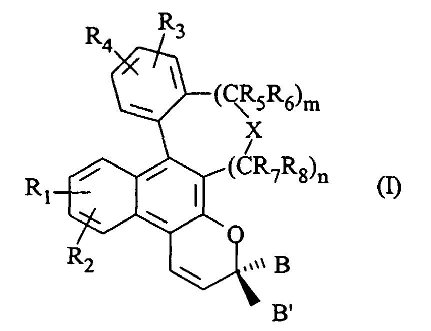 (I):
worin
n und m unabhängig voneinander 0, 1 oder 2 bedeuten,
die Reste R₁, R₂, R₃ und R₄ jeweils unabhängig voneinander einen Substituenten darstellen, ausgewählt aus
der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome aufweisen kann, einem (C₁-C₆)-Alkoxyrest, einer Hydroxygruppe, einer Trifluormethylgruppe, Brom, Chlor und Fluor;
der Gruppe β, bestehend aus einem un-, mono- oder disubstituiertem Phenyl-, Phenoxy-, Benzyl-, Benzyloxy-, Naphthyl- oder Naphthoxyrest, wobei die Substituenten aus der Gruppe α und Phenyl ausgewählt sein können;
der Gruppe γ, worin die Reste R₁ und R₂ bzw. R₃ und R₄ jeweils eine an den aromatischen Ring gebundene -A-(CH₂)ₖ-D- Gruppe oder -A-(C(CH₃)₂)ₖ-D- Gruppe mit k = 1 oder 2 bilden, wobei A und B unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann;
die Reste R₅, R₆, R₇ und R₈ jeweils unabhängig voneinander aus der Gruppe α und Phenyl ausgewählt sind oder die Reste R₅ und R₆ zusammen mit dem Rest R₃ des direkt benachbarten Benzoringes einen daran annellierten, un-, mono- oder disubstituierten Benzo- oder Pyridoring bilden, dessen Substituenten aus der Gruppe α und Phenyl ausgewählt sein können, oder, wenn m bzw. n 2 bedeuten, die direkt benachbarten Reste R₅ und R₆ zweier benachbarter CR₅R₆-Einheiten bzw. die direkt benachbarten Reste R₇ und R₈ zweier benachbarter CR₇R₈-Einheiten miteinander einen annellierten, un-, mono- oder disubstituierten Benzo- oder Pyridoring bilden, dessen Substituenten aus der Gruppe α und Phenyl ausgewählt sein können, oder die Reste R₅ und R₆ und/oder die Reste R₇ und R₈ zusammen einen (C₃-C₇)-Cycloalkylrest darstellen, der ein oder mehrere Heteroatome, wie beispielsweise Sauerstoff oder Schwefel, aufweisen kann, wobei an diesen Cycloalkylrest ein Benzoring annelliert sein kann;
X aus O, S oder CR₉R₁₀ ausgewählt ist, wobei die Reste R₉ und R₁₀ unabhängig voneinander aus der Gruppe α und Phenyl ausgewählt sind oder die Reste R₉ und R₁₀ zusammen einen (C₃-C₇)-Cycloalkylrest darstellen, der ein oder mehrere Heteroatome aufweisen kann, oder die Reste R₉ und R₁₀ zusammen mit den Resten R₅ und R₆ bzw. R₇ und R₈ einer direkt benachbarten CR₅R₆-Einheit bzw. CR₇R₈-Einheit für einen an die X-C(R₅R₆) bzw. X-C(R₇R₈)-Bindung annellierten, un-, mono- oder disubstituierten Benzo- oder Pyridoring stehen können, dessen Substituenten aus der Gruppe α und Phenyl ausgewählt sein können; mit der Maßgabe, dass X nicht CR₉R₁₀ sein kann, wenn m und n beide 0 sind;
B und B' unabhängig voneinander aus einer der folgenden Gruppen a), b), c) oder d) ausgewählt sind, wobei
a) mono-, di- und trisubstituierte Arylreste sind, wobei der Arylrest Phenyl oder Naphthyl ist;
b) unsubstituierte, mono- und disubstituierte Heteroarylreste sind, wobei der Heteroarylrest Pyridyl, Furanyl, Benzofuran-2-yl, Benzofuran-3-yl, Dibenzofuranyl, Thienyl, Benzothien-2-yl, Benzothien-3-yl, oder Dibenzothienyl ist;
wobei die Substituenten der Aryl- oder Heteroarylreste in a) und b) solche sind, ausgewählt aus den Gruppen α, β oder γ oder einer un-, mono- oder disubstituierten Aminogruppe, wobei die Aminsubstituenten aus einem (C₁-C₆)-Alkylrest, einem (C₃-C₇)-Cycloalkylrest, einem unsubstituierten oder mit einem oder mehreren Substituenten aus der Gruppe α substituierten Phenyl- oder Benzylrest ausgewählt sein können, einer N-Morpholingruppe, einer N-Thiomorpholingruppe, einer N-Piperidingruppe, einer N-Azacycloheptangruppe, einer N-Piperazingruppe, einer N-(N'-(C₁-C₆-Alkyl)piperazingruppe, einer N-Pyrrolidingruppe, einer N-Imidazolidingruppe, einer N-Pyrazolidingruppe, einer N-Aziridingruppe, einer N-Azetidingruppe, einer N-Indolingruppe, einer N-Carbazolgruppe, einer N-Phenothiazingruppe, einer N-Phenazingruppe, einer N-Phenoxazingruppe, einer N-Tetrahydrochinolingruppe oder einer N-Tetrahydroisochinolingruppe;
c) Struktureinheiten mit den folgenden Formeln (V) und (W) sind: 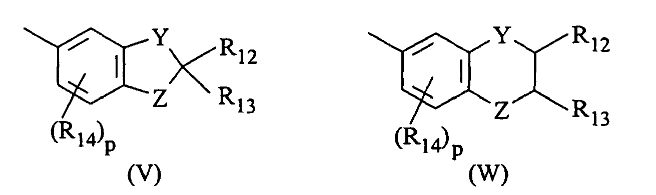 worin
Y und Z unabhängig voneinander für O, S, CH₂, CMe₂, NH, NPh oder N(C₁-C₆)-Alkyl stehen, die Reste R₁₂ und R₁₃ unabhängig voneinander Wasserstoff oder einen (C₁-C₆)-Alkylrest darstellen und der Rest R₁₄ ein Substituent aus der Gruppe α ist, wobei p 1, 2, oder 3 ist,
oder
d) B und B' miteinander einen un-, mono- oder disubstituierten 9,10-Dihydroanthracen-, Fluoren-, Thioxanthen- oder Xanthen-9-ylidenrest, Benzo[b]flouren-11-ylidenrest, 5H-Dibenzo[a,c]cyclohepten-, Dibenzosuberon- oder 5H-Dibenzo[a,c]cyclooctan-5-ylidenrest oder einen gesättigten Kohlenwasserstoffrest bilden, der C₃-C₁₂ spiro-monozyklisch, C₇-C₁₂ spiro-bizyklisch und/oder C₇-C₁₂ spiro-trizyklisch ist, wobei die Substituenten der ungesättigten Cyclen aus der Gruppe α ausgewählt sind.

2. Photochrome Benzo[f]chromene nach Anspruch 1, wobei der gemäß Formel (I) in h-Position des Benzo[f]chromen-Systems annellierte Cyclus bzw. Hetero- cyclus ein Fünfring, Sechsring oder Siebenring ist.

3. Photochrome Benzo[f]chromene nach Anspruch 1 oder 2, welche die nachfolgenden allgemeinen Formeln (II), (III) bzw. (IV) aufweisen: 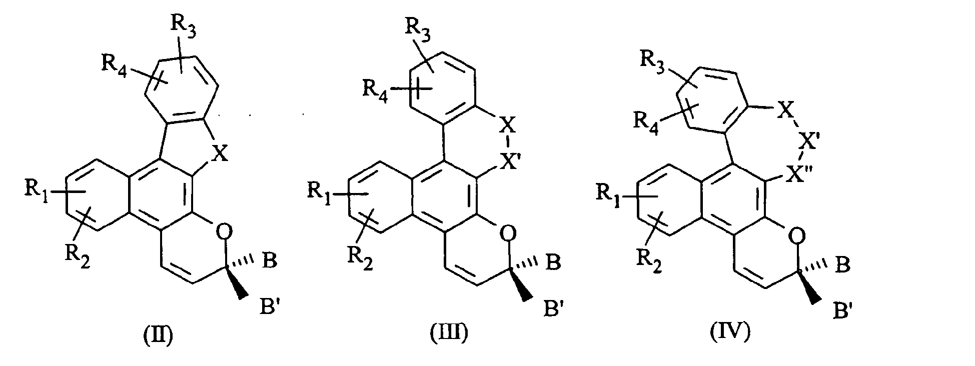 worin B, B', R₁, R₂, R₃ und R₄ wie vorgenannt definiert sind und in Formel (II) X = O oder S ist;
in Formel (III) X und X' unabhängig voneinander aus O, S oder CR₉R₁₀ ausgewählt sind, mit der Maßgabe, dass mindestens eines von beiden CR₉R₁₀ ist;
in Formel (IV) X, X' und X" unabhängig voneinander aus O, S oder CR₉R₁₀ ausgewählt sind, mit der Maßgabe, dass, wenn X' = O oder S ist, X und X" = CR₉R₁₀ sind.

4. Photochrome Benzo[f]chromene nach einem der Ansprüche 1 bis 3, wobei die Reste R₁, R₂, R₃ und R₄ aus den Gruppen α und β ausgewählt sind.

5. Photochrome Benzo[f]chromene nach einem der Ansprüche 1 bis 4, wobei die Reste R₅, R₆, R₇ und R₈ jeweils unabhängig voneinander aus der Gruppe α ausgewählt sind.

6. Photochrome Benzo[f]chromene nach einem der Ansprüche 1 bis 5, wobei in den vorstehend aufgeführten Formeln (I), (II), (III) bzw. (IV) B und B' unabhängig voneinander für mono-, di- oder trisubstituierte Arylreste stehen, wobei der Arylrest jeweils ein Phenylrest oder ein Naphthylrest ist.

7. Photochrome Benzo[f]chromene nach einem der vorhergehenden Ansprüche, welche
2-(4-Methoxyphenyl)-2-phenyl-2H-benzofurano[1,2-h]benzo[f]chromen,
2-(4-Methoxyphenyl)-2-phenyl-2H-benzothiopheno[1,2-h]benzo[f]chromen,
2-(4-Methoxyphenyl)-2-phenyl-2H-13,14-dihydro-naphtho[1,2h]-benzo[f]chromen,
7-Methoxy-2-(4-methoxyphenyl)-2-phenyl-2H-13,14-dihydro-naphtho[1,2-h]-benzo[f]chromen,
11-Methoxy-2-(4-methoxyphenyl)-2-phenyl-2H-13,14-dihydro-naphtho[1,2-h]-benzo[f]chromen,
2-(4-Methoxyphenyl)-2-phenyl-2H,13H-chromeno[1,2-h]benzo[f]chromen,
2-(4-Methoxyphenyl)-2-phenyl-2H,14H-chromeno[1,2-h]benzo[f]chromen bzw.
7-Methoxy-2-(4-methoxyphenyl)-2-phenyl-2H-benzocycloheptano-[1,2-h]-benzo[f]chromen
sind.

8. Verwendung der photochromen Benzo[f]chromene nach einem der Ansprüche 1 bis 7 in Kunststoffmaterialien.

9. Verwendung nach Anspruch 8, wobei das Kunststoffmaterial eine ophthalmische Linse ist.

## Claims

1. Photochromic h-annelated benzo[f]chromenes with the general formula (I): 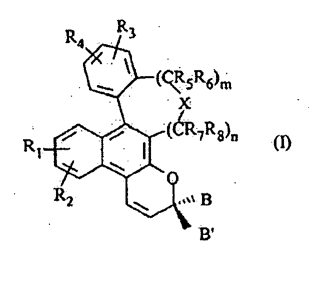 wherein
n and m, independently of one another, represent 0, 1 or 2,
residues R₁, R₂, R₃ and R₄, respectively independently of one another, represent a substituent selected from
group α consisting of a hydrogen atom, a (C₁-C₆) alkyl residue, a (C₁-C₆) thioalkyl residue, a (C₃-C₇) cycloalkyl residue, which can have one or more heteroatoms, a (C₁-C₆) alkoxy residue, a hydroxy group, a trifluoromethyl group, bromine, chlorine and fluorine;
group β consisting of an un-, mono- or disubstituted phenyl, phenoxy, benzyl, benzyloxy, naphthyl or naphthoxy residue, wherein the substituents can be selected from group α and phenyl;
group γ, wherein the residues R₁ and R₂ or R₃ and R₄ respectively form an -A-(CH₂)ₖ-D- group or -A-(C(CH₃)₂)ₖ-D- group bonded to the aromatic ring with k = 1 or 2, wherein A and B, independently of one another, are selected from oxygen, sulphur, CH₂, C(CH₃)₂ or C(C₆H₅)₂, and wherein a benzo ring can in turn be annelated onto this -A-(CH₂)ₖ-D- group;
residues R₅, R₆, R₇ and R₈, respectively independently of one another, are selected from group α and phenyl or residues R₅ and R₆ together with residue R₃ of the directly adjacent benzo ring form an un-, mono- or disubstituted benzo or pyrido ring annelated onto said directly adjacent benzo ring, and the substituents of said benzo or pyrido ring can be selected from group α and phenyl, or if m or n represent 2, the directly adjacent residues R₅ and R₆ of two adjacent CR₅R₆ units or the directly adjacent residues R₇ and R₈ of two adjacent CR₇R₈ units together form an annelated un-, mono- or disubstituted benzo or pyrido ring, the substituents of which can be selected from group α and phenyl, or residues R₅ and R₆ and/or residues R₇ and R₈ together represent a (C₃-C₇) cycloalkyl residue, which can have one or more heteroatoms such as e.g. oxygen or sulphur, wherein a benzo ring can be annelated onto this cycloalkyl residue;
X is selected from O, S or CR₉R₁₀, wherein residues R₉ and R₁₀, independently of one another, are selected from group α and phenyl or residues R₉ and R₁₀ together represent a (C₃-C₇) cycloalkyl residue, which can have one or more heteroatoms, or residues R₉ and R₁₀ together with residues R₅ and R₆ or R₇ and R₈ of a directly adjacent CR₅R₆ unit or CR₇R₈ unit can stand for an un-, mono- or disubstituted benzo or pyrido ring annelated to the X-C(R₅R₆) or X-C(R₇R₈) bond, and the substituents of said benzo or pyrido ring can be selected from group α and phenyl; on condition that X cannot be CR₉R₁₀ when m and n are both 0;
B and B', independently of one another, are selected from one of the following groups a), b), c) or d), wherein
a) are mono-, di- and trisubstituted aryl residues, wherein the aryl residue is phenyl or naphthyl;
b) are unsubstituted, mono- and disubstituted heteroaryl residues, wherein the heteroaryl residue is pyridyl, furanyl, benzofuran-2-yl, benzofuran-3-yl, dibenzofuranyl, thienyl, benzothien-2-yl, benzothiene-3-yl or dibenzothienyl; wherein the substituents of the aryl or heteroaryl residues in a) and b) are such, selected from groups α, β or γ or an un-, mono- or disubstituted amino group, wherein the amino substituents can be selected from a (C₁-C₆) alkyl residue, a (C₃-C₇) cycloalkyl residue, an unsubstituted phenyl or benzyl residue or a phenyl or benzyl residue substituted with one or more substituents from group α, an N-morpholine group, an N-thiomorpholine group, an N-piperidine group, an N-azacycloheptane group, an N-piperazine group, an N-(N'-(C₁-C₆-alkyl)piperazine group, an N-pyrrolidine group, an N-imidazoline group, an N-pyrazolidine group, an N-aziridine group, an N-azetidine group, an N-indoline group, an N-carbazole group, an N-phenothiazine group, an N-phenazine group, an N-phenoxazine group, an N-tetrahydroquinoline group or an N-tetrahydroisoquinoline group;
c) structural elements with the following formulae (V) and (W) are: 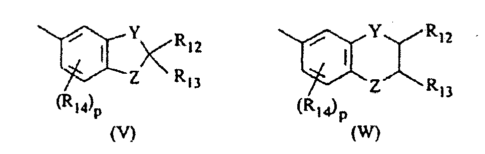 wherein
Y and Z, independently of one another, stand for O, S, CH₂, CMe₂, NH, NPh or N(C₁-C₆) alkyl, residues R₁₂ and R₁₃, independently of one another, represent hydrogen or a (C₁-C₆) alkyl residue and residue R₁₄ is a substituent from group α, wherein p is 1, 2 or 3,
or
d) B and B' together form an un-, mono- or disubstituted 9,10-dihydroanthracene, fluorene, thioxanthene or xanthen-9-ylidene residue, benzo[b]fluoren-11-ylidene residue, 5H-dibenzo[a,c]cycloheptene, dibenzosuberone or 5H-dibenzo[a,c]cyclooctane-5-ylidene residue or a saturated hydrocarbon residue, which is C₃-C₁₂ spiro-monocyclic, C₇-C₁₂ spiro-bicyclic and/or C₇-C₁₂ spiro-tricyclic, wherein the substituents of the unsaturated cycles are selected from group α.

2. Photochromic benzo[f]chromenes according to claim 1, wherein the cycle or heterocycle annelated in h-position of the benzo[f]chromene system according to formula (I) is a five-membered ring, six-membered ring or seven-membered ring.

3. Photochromic benzo[f]chromenes according to claim 1 or 2, which have the following general formulae (II), (III) or (IV): 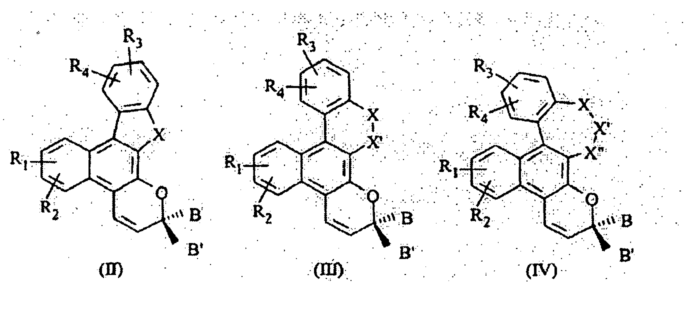 wherein B, B', R₁, R₂, R₃ and R₄ are as defined above and
in formula (II) X = O or S;
in formula (III) X and X', independently of one another, are selected from O, S or CR₉R₁₀, with the provision that at least one of the two is CR₉R₁₀;
in formula (IV) X, X' and X", independently of one another, are selected from O, S or CR₉R₁₀, with the provision that when X' = O or S, X and X" = CR₉R₁₀.

4. Photochromic benzo[f]chromenes according to one of claims 1 to 3, wherein residues R₁, R₂, R₃ and R₄ are selected from groups α and β.

5. Photochromic benzo[f]chromenes according to one of claims 1 to 4, wherein residues R₅, R₆, R₇ and R₈, respectively independently of one another, are selected from group α.

6. Photochromic benzo[f]chromenes according to one of claims 1 to 5, wherein in the above-specified formulae (I), (II), (III) or (IV) B and B', independently of one another, stand for mono-, di- or trisubstituted aryl residues, wherein the aryl residue is respectively a phenyl residue or a naphthyl residue.

7. Photochromic benzo[f]chromenes according to one of the preceding claims, which are
2-(4-methoxyphenyl)-2-phenyl-2H-benzofurano[1,2-h]benzo[f]chromenes,
2-(4-methoxyphenyl)-2-phenyl-2H-benzothiopheno[1,2-h]benzo[f]chromenes,
2-(4-methoxyphenyl)-2-phenyl-2H-13,14-dihydro-naphtho[1,2-h]benzo[f]chromenes,
7-methoxy-2-(4-methoxyphenyl)-2-phenyl-2H-13,14-dihydro-naphtho[1,2-h]-benzo[f]chromenes,
11-methoxy-2-(4-methoxyphenyl)-2-phenyl-2H-13,14-dihydro-naphtho[1,2-h]-benzo[f]chromenes,
2-(4-methoxyphenyl)-2-phenyl-2H,13H-chromeno[1,2-h]benzo[f]chromenes,
2-(4-methoxyphenyl)-2-phenyl-2H,14H-chromeno[1,2-h]benzo[f]chromenes, or
7-methoxy-2-(4-methoxyphenyl)-2-phenyl-2H-benzocycloheptano-[1,2-h]-benzo[f]chromenes.

8. Use of the photochromic benzo[f]chromenes according to one of claims 1 to 7 in plastic materials.

9. Use according to claim 8, wherein the plastic material is an ophthalmic lens.

## Revendications

1. Benzo[f]chromènes photochromes, condensés en h, ayant pour formule générale (I) : 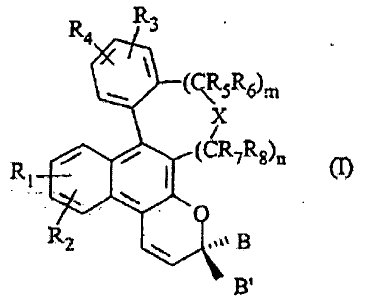 dans laquelle
n et m, indépendamment l'un de l'autre, représentent 0, 1 ou 2,
les radicaux R₁, R₂, R₃ et R₄ représentent, chacun indépendamment les uns des autres, un substituant qui est choisi
dans le groupe α constitué par un atome d'hydrogène, un radical alkyle (en C₁-C₆), un radical thioalkyle (en C₁-C₆), un radical cycloalkyle (en C₃-C₇), qui peut comporter un ou plusieurs hétéroatomes, un radical alcoxy (en C₁-C₆), un groupe hydroxy, un groupe trifluorométhyle, le brome, le chlore et le fluor ;
dans le groupe β constitué d'un radical phényle, phénoxy, benzyle, benzyloxy, naphtyle ou naphtoxy non substitué, mono- ou disubstitué, les substituants pouvant être choisis entre le groupe α et le phényle ;
dans le groupe γ, dans lequel les radicaux R₁ et R₂ ou R₃ et R₄ forment, respectivement, un groupe -A-(CH₂)ₖ-D ou -A-(C(CH₃)₂)ₖ-D lié au cycle aromatique, avec k = 1 ou 2, où A et B sont choisis indépendamment l'un de l'autre parmi oxygène, soufre, CH₂, C(CH₃)₂ ou C(C₆H₅)₂, et où un cycle benzénique peut être condensé de nouveau à ce groupe -A-(CH₂)ₖ-D ;
les radicaux R₅, R₆, R₇ et R₈ sont choisis, respectivement indépendamment les uns des autres, entre le groupe α et le phényle, ou les radicaux R₅ et R₆ forment, conjointement avec le radical R₃ du cycle benzénique directement adjacent, un cycle benzénique ou pyridinique non substitué, mono- ou disubstitué, condensé à celui-ci, dont les substituants peuvent être choisis entre le groupe α et le phényle, ou, si m ou n représente 2, les radicaux R₅ et R₆ directement adjacents de deux unités CR₅R₆ adjacentes ou les radicaux R₇ et R₈ directement adjacents de deux unités CR₇R₈ adjacentes forment ensemble un cycle benzénique ou pyridinique non substitué, mono- ou disubstitué condensé, dont les substituants peuvent être choisis entre le groupe α et le phényle, ou les radicaux R₅ et R₆ et/ou les radicaux R₇ et R₈ représentent conjointement un radical cycloalkyle (en C₃-C₇), qui peut comporter un ou plusieurs hétéroatomes, comme par exemple l'oxygène ou le soufre, un cycle benzénique pouvant être condensé à ce radical cycloalkyle ;
X est choisi parmi O, S ou CR₉R₁₀, les radicaux R₉ et R₁₀ étant choisis, indépendamment les uns des autres, entre le groupe α et le phényle ou les radicaux R₉ et R₁₀ représentent conjointement un radical cycloalkyle (en C₃-C₇), qui peut comporter un ou plusieurs hétéroatomes, ou les radicaux R₉ et R₁₀ peuvent représenter conjointement avec les radicaux R₅ et R₆ ou R₇ et R₈ d'une unité CR₅R₆ ou CR₇R₈ directement adjacente un cycle benzénique ou pyridinique non substitué, mono- ou disubstitué condensé à la liaison X-C(R₅R₆) ou X-C(R₇R₈) dont les substituants peuvent être choisis entre le groupe α et le phényle ; à la condition que X ne puisse être CR₉R₁₀, quand m et n sont tous deux égaux à 0 ;
B et B', indépendamment l'un de l'autre, sont choisis dans l'un des groupes suivants a), b), c) ou d), où
a) constitue les radicaux aryles mono-, di- ou trisubstitués, le radical aryle étant le phényle ou le napthyle ;
b) constitue les radicaux hétéroaryles non substitués, mono- ou disubstitués, le radical hétéroaryle étant pyridyle, furanyle, benzofuran-2-yle, benzofuran-3-yle, dibenzofuranyle, thiényle, benzothién-2-yle, benzothién-3-yle ou dibenzothiényle ;
où les substituants des radicaux aryles ou hétéroaryles dans a) et b) sont ceux choisis dans les groupes α, β ou γ ou parmi un groupe amino non substitué, monosubstitué ou disubstitué, les substituants amines pouvant être choisis parmi un radical alkyle (en C₁-C₆), un radical cycloalkyle (en C₃-C₇), un radical phényle ou benzyle non substitué ou substitué avec ou plusieurs substituants du groupe α, parmi un groupe N-morpholine, un groupe N-thiomorpholine, un groupe N-pipéridine, un groupe N-azacycloheptane, un groupe N-pipérazine, un groupe N-(N'-(alkyle en C₁-C₆)pipérazine), un groupe N-pyrrolidine, un groupe N-imidazolidine, un groupe N-pyrazolidine, un groupe N-aziridine, un groupe N-azétidine, un groupe N-indoline, un groupe N-carbazole, un groupe N-phénothiazine, un groupe N-phénazine, un groupe N-phénoxazine, un groupe N-tétrahydroquinoline ou un groupe N-tétrahydro-isoquinoline ;
c) constitue les motifs structurels ayant les formules (V) et (W) suivantes : 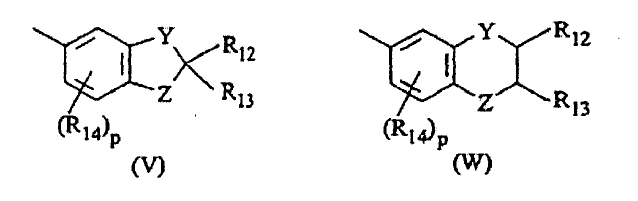 Y et Z, indépendamment l'un de l'autre, représentent O, S, CH₂, CMe₂, NH, NPh ou N-alkyle(en C₁-C₆), les radicaux R₁₂ et R₁₃, représentent, indépendamment l'un de l'autre, hydrogène ou un radical alkyle(en C₁-C₆), et le radical R₁₄ est un substituant provenant du groupe α, où p est égal à 1, 2 ou 3,
ou dans laquelle
d) B et B' représentent conjointement un radical 9,10-dihydroanthracèn-, fluorèn-, thioxanthèn- ou xanthèn-9-ylidène, un radical benzo[b]fluorèn-11-ylidène, un radical 5H-dibenzo[a,c]cycloheptèn-, dibenzosubéron- ou 5H-dibenzo[a,c]cyclooctan-5-ylidène, tous non substitués, mono- ou disubstitués, ou un radical hydrocarbure saturé, qui est spiromonocyclique en C₃-C₁₂, spirobicyclique en C₇-C₁₂ et/ou spirotricyclique en C₇-C₁₂, où les substituants des cycles insaturés sont choisis dans le groupe α.

2. Benzo[f]chromènes photochromes selon la revendication 1, dans lesquels le cycle ou l'hétérocycle condensé selon la formule (I) en position h du système benzo[f]chromène est un cycle à cinq, six ou sept chaînons.

3. Benzo[f]chromènes photochromes selon la revendication 1 ou 2, présentant les formules générales (II), (III) ou (IV) suivantes : 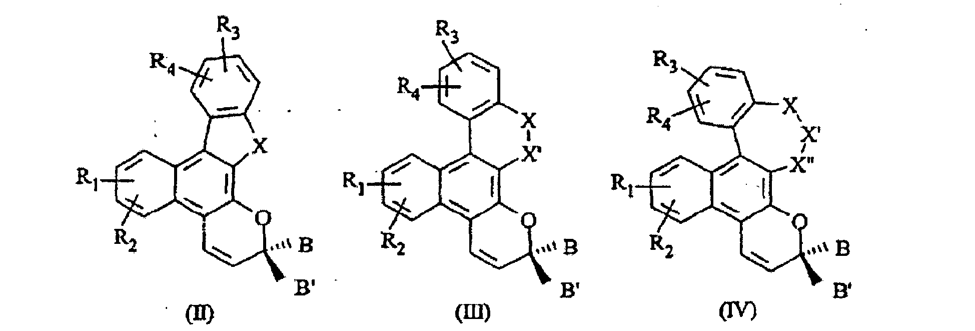 dans lesquelles B, B', R₁, R₂, R₃ et R₄ sont tels que définis précédemment, et
dans la formule (II), X = O ou S ;
dans la formule (III), X et X' sont choisis, indépendamment l'un de l'autre, parmi O, S ou CR₉R₁₀, à la condition qu'au moins l'un des deux soit CR₉R₁₀,
dans la formule (IV), X, X' et X'' sont choisis, indépendamment les uns des autres, parmi O, S ou CR₉R₁₀, à la condition que si X' = O ou S, X et X'' CR₉R₁₀.

4. Benzo[f]chromènes photochromes selon l'une des revendications 1 à 3, dans lesquels R₁, R₂, R₃ et R₄ sont choisis dans les groupes α et β.

5. Benzo[f]chromènes photochromes selon l'une des revendications 1 à 4, dans lesquels les radicaux R₅, R₆, R₇ et R₈ sont choisis, respectivement indépendamment les uns des autres, dans le groupe α.

6. Benzo[f]chromènes photochromes selon l'une des revendications 1 à 5, dans lesquels, dans les formules (I), (II), (III) ou (IV) indiquées ci-dessus, B et B' représentent, indépendamment l'un de l'autre, des radicaux aryles mono-, di- ou trisubstitués, le radical aryle étant respectivement un radical phényle ou un radical naphtyle.

7. Benzo[f]chromènes photochromes selon l'une des revendications précédentes, qui sont :
le 2-(4-méthoxyphényl)-2-phényl-2H-benzofurano[1,2-h]benzo[f]chromène,
le 2-(4-méthoxyphényl)-2-phényl-2H-benzothiophéno[1,2-h]benzo[f]chromène,
le 2-(4-méthoxyphényl)-2-phényl-2H-13,14-dihydro-naphto[1,2-h]benzo[f]chromène,
le 7-méthoxy-2-(4-méthoxyphényl)-2-phényl-2H-13,14-dihydro-naphto-[1,2-h]benzo[f]chromène,
le 11-méthoxy-2-(4-méthoxyphényl)-2-phényl-2H-13,14-dihydro-naphto-[1,2-h]benzo[f]chromène,
le 2-(4-méthoxyphényl)-2-phényl-2H,13H-chromèno[1,2-h]benzo[f]chromène,
le 2-(4-méthoxyphényl)-2-phényl-2H,14H-chromèno[1,2-h]benzo[f]chromène, ou
le 7-méthoxy-2-(4-méthoxyphényl)-2-phényl-2H-benzocyclo-heptano-[1,2-h]benzo[f]chromène.

8. Utilisation des benzo[f]chromènes photochromes selon l'une des revendications 1 à 7 dans des matières plastiques.

9. Utilisation selon la revendication 8, dans laquelle la matière plastique est une lentille ophtalmique.
